# EUROPEAN PATENT APPLICATION

(11) **EP 4 524 481 A1**
(43) Date of publication of application: **19.03.2025**
(21) Application number: 23831710.1
(22) Date of filing: 03.05.2023
(51) Int. Cl.: F24F 1/0071, F24F 8/22, A61L 2/10, F24F 1/0018, F24F 1/0063, F24F 13/22, F24F 1/0076, F24F 13/20

(54) **STERILIZATION DEVICE AND AIR CONDITIONER HAVING SAME**

(30) Priority: 01.07.2022 KR 20220081520; 29.08.2022 KR 20220108753
(71) Applicant: Samsung Electronics Co., Ltd., Suwon-si, Gyeonggi-do 16677 (KR)
(72) Inventor: LEE, Woonghee, Suwon-si Gyeonggi-do 16677 (KR); AHN, Soonwon, Suwon-si Gyeonggi-do 16677 (KR); YOON, Joonho, Suwon-si Gyeonggi-do 16677 (KR); LEE, Wonhee, Suwon-si Gyeonggi-do 16677 (KR); LEE, Jangjung, Suwon-si Gyeonggi-do 16677 (KR)
(74) Representative: Walaski, Jan Filip
(86) International application number: PCT/KR2023/006028
(87) International publication number: WO 2024/005346

(57) **Abstract**

An air conditioner according to an embodiment of the disclosure includes a housing having an inlet and an outlet, a heat exchanger disposed within the housing to heat exchange with air drawn in through the inlet, a blower fan configured to flow heat exchanged air to allow the air heat exchanged by the heat exchanger to be discharged through the outlet, and a sterilization device configured to irradiate light toward the blower fan. The sterilization device includes a first light-emitting diode (LED) including a first irradiation surface, and a second LED including a second irradiation surface. The first LED is arranged such that the first irradiation surface faces a first direction, and the second LED is arranged such that the second irradiation surface faces a second direction different from the first direction.

## Description

### [Technical Field]

The disclosure relates to a sterilization device and an air conditioner having the same.

### [BACKGROUND ART]

In general, air conditioners are devices that include a compressor, a condenser, an expansion valve, an evaporator, a blower fan, and the like, and use a refrigeration cycle to regulate the temperature, humidity, airflow, and the like in a room. Air conditioners may be categorized as split type, which has an indoor unit placed indoors and an outdoor unit placed outdoors, or integrated type, which has both indoor unit and outdoor units placed in a single housing.

The indoor unit of an air conditioner may be mounted on the ceiling of a room. The indoor unit of an air conditioner may draw in air from the room, heat exchange the drawn-in air with a heat exchanger, and then discharge the heat-exchanged air.

The air conditioner includes a heat exchanger that exchanges heat between refrigerant and air, a fan that blows air, and a motor that drives the fan, and cools or heats the room.

### [DISCLOSURE]

### [TECHNICAL PROBLEM]

Provided are a sterilization device having a sterilizing function and an air conditioner having the same.

Further, provided are a sterilization device having an improved sterilizing function by irradiating light towards a blower fan, and an air conditioner having the same.

Technical tasks to be achieved in this document are not limited to the technical tasks mentioned above, and other technical tasks not mentioned will be clearly understood by those skilled in the art from the description below.

### [TECHNICAL SOLUTION]

According to an embodiment of the disclosure, an air conditioner includes a housing having an inlet and an outlet, a heat exchanger disposed within the housing to heat exchange with air drawn in through the inlet, a blower fan configured to flow heat exchanged air to allow the air heat exchanged by the heat exchanger to be discharged through the outlet, and a sterilization device configured to irradiate light toward the blower fan. The sterilization device includes a first light-emitting diode (LED) including a first irradiation surface, and a second LED including a second irradiation surface. The first LED is arranged such that the first irradiation surface faces a first direction, and the second LED is arranged such that the second irradiation surface faces a second direction different from the first direction.

The sterilization device may further include a third LED spaced apart from the first LED 120a and including a third irradiation surface, wherein the third LED may be arranged such that the third irradiation surface faces the first direction.

The sterilization device may further include a blocking protrusion configured to prevent light from being exposed to an outside of the housing.

The blocking protrusion may include a first blocking protrusion extending toward the first direction, and a second blocking protrusion extending toward the second direction.

The housing may further include a lower panel in which the inlet and the outlet are formed, the first blocking protrusion may be disposed at a lower end of the first irradiation surface to prevent light irradiated from the first LED from penetrating the lower panel, and the second blocking protrusion may be disposed at a lower end of the second irradiation surface to prevent light irradiated from the second LED from penetrating the lower panel.

The heat exchanger may be arranged to surround the blower fan, and the sterilization device may be disposed between the heat exchanger and the blower fan.

The first LED may be arranged to be inclined toward the blower fan as the first LED is further away from the second LED.

The sterilization device may further include a case configured to accommodate the first LED and the second LED, and a holder configured to support the case.

The case may further include a case hinge configured to allow the second LED to rotate relative to the first LED.

The air conditioner may further include a sterilization coupling portion disposed within the housing and coupled to the holder in order to support the sterilization device, wherein the holder includes a first frame couplable to the case, and a second frame connected to the first frame and couplable to the sterilization coupling portion.

The air conditioner may further include a drain pan arranged on a lower portion of the heat exchanger and storing condensed water, wherein the sterilization coupling portion is arranged at the drain pan.

The air conditioner may further include a bell mouth configured to guide air drawn in through the inlet to the blower fan, wherein the sterilization coupling portion is arranged at the bell mouth.

The first frame may include a first hole and a second hole spaced apart from the first hole, wherein the case is connectable to the first hole or the second hole.

The holder may further include a holder hinge configured to allow the first frame to rotate relative to the second frame.

The holder may further include a cable receiving portion configured to secure a cable that supplies power to the first LED and the second LED.

According to an embodiment of the disclosure, a sterilization device includes a case, a first light-emitting diode (LED), and a second LED, wherein the case includes a first surface on which the first LED is seated and a second surface on which the second LED is seated, wherein an imaginary first extension line orthogonal to the first surface is arranged to intersect an imaginary second extension line orthogonal to the second surface.

The sterilization device may further include a third LED spaced apart from the first LED, and the third LED may be seated on the first surface.

The sterilization device may further include a first blocking protrusion extending along the first extension line and a second blocking protrusion extending along the second extension line.

The case may further include a case hinge configured to allow the second LED to rotate relative to the first LED.

The sterilization device may further include a holder configured to support the case, wherein the holder may include a first frame couplable to the case and a second frame connected to the first frame by a hinge such that the first frame is rotatably connected to the case.

### [ADVANTAGEOUS EFFECTS]

An aspect of the present disclosure provides the sterilization device having a sterilizing function and an air conditioner having the same.

Further, an aspect of the present disclosure provides the sterilization device having an improved sterilizing function by irradiating light toward the blower fan, and an air conditioner having the same.

Effects in accordance with the spirit of the present disclosure are not limited to those mentioned above, and other effects not mentioned will be apparent to those skilled in the art to which the present disclosure belongs from the following description.

### [DESCRIPTION OF DRAWINGS]

FIG. 1 is a perspective view illustrating an air conditioner according to an embodiment of the present disclosure.
FIG. 2 is an exploded perspective view illustrating a configuration of the air conditioner according to an embodiment of the present disclosure.
FIG. 3 is a cross-sectional view illustrating a cross-section of the air conditioner according to an embodiment of the present disclosure.
FIG. 4 is a front perspective view illustrating a sterilization device according to an embodiment of the present disclosure.
FIG. 5 is a rear perspective view of the sterilization device according to an embodiment of the present disclosure.
FIG. 6 is an exploded front perspective view illustrating a configuration of the sterilization device according to an embodiment of the present disclosure.
FIG. 7 is an exploded rear perspective view illustrating the configuration of the sterilization device according to an embodiment of the present disclosure.
FIG. 8 is a top view illustrating light irradiated from the sterilization device according to an embodiment of the present disclosure.
FIG. 9 is a view illustrating the sterilization device according to an embodiment of the present disclosure irradiating light toward a blower fan.
FIG. 10 is a perspective view illustrating an air conditioner according to an embodiment of the present disclosure.
FIG. 11 is an exploded perspective view illustrating a configuration of the air conditioner according to an embodiment of the present disclosure.
FIG. 12 is a cross-sectional view illustrating a cross-section of the air conditioner according to an embodiment of the present disclosure.
FIG. 13 is a front perspective view illustrating a sterilization device according to an embodiment of the present disclosure.
FIG. 14 is a rear perspective view illustrating the sterilization device according to an embodiment of the present disclosure.
FIG. 15 is an exploded front perspective view illustrating a configuration of the sterilization device according to an embodiment of the present disclosure.
FIG. 16 is an exploded rear perspective view illustrating the configuration of the sterilization device according to an embodiment of the present disclosure.
FIG. 17 is a front perspective view illustrating a sterilization device according to an embodiment of the present disclosure.
FIG. 18 is a top view illustrating light irradiated from the sterilization device according to an embodiment of the present disclosure.
FIG. 19 is a top view of the sterilization device of FIG. 18 being rotated.

### [MODES OF THE INVENTION]

Embodiments described in the disclosure and configurations shown in the drawings are merely examples of the embodiments of the disclosure and may be modified in various different ways at the time of filing of the present application to replace the embodiments and drawings of the disclosure.

In connection with the description of the drawings, similar reference numerals may be used for similar or related components.

Also, the terms used herein are used to describe the embodiments and are not intended to limit and/or restrict the disclosure. The singular forms "a," "an" and "the" are intended to include the plural forms as well, unless the context clearly indicates otherwise. In this disclosure, the terms "including", "having", and the like are used to specify features, figures, steps, operations, elements, components, or combinations thereof, but do not preclude the presence or addition of one or more of the features, figures, steps, operations, elements, components, or combinations thereof.

It will be understood that, although the terms "first", "second", "primary", "secondary", etc., may be used herein to describe various elements, but elements are not limited by these terms. These terms are only used to distinguish one element from another element. For example, without departing from the scope of the disclosure, a first element may be termed as a second element, and a second element may be termed as a first element. The term of "and/or" includes a plurality of combinations of relevant items or any one item among a plurality of relevant items.

As used herein, the terms "front", "rear", "upper", "lower", "left", "right", and the like are defined with reference to the drawings and are not intended to limit the shape and location of each component.

Hereinafter, various embodiments of the disclosure will be described in detail with reference to the accompanying drawings.

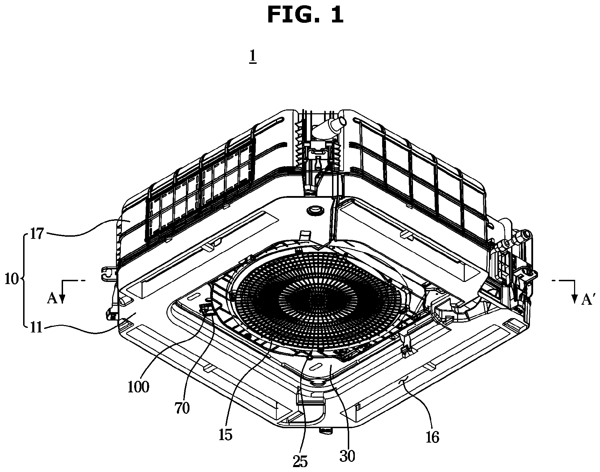

FIG. 1 is a perspective view showing an air conditioner according to an embodiment of the present disclosure.

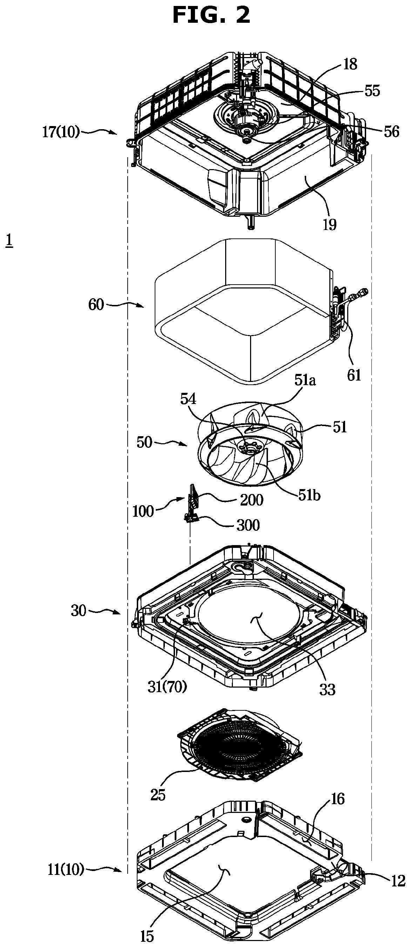

FIG. 2 is an exploded perspective view showing a configuration of the air conditioner according to an embodiment of the present disclosure.

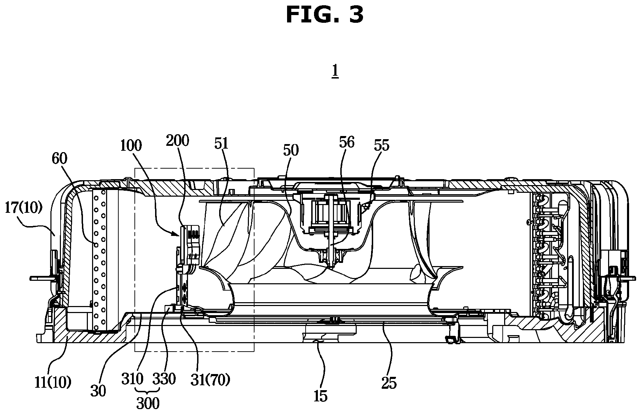

FIG. 3 is a cross-sectional view showing a cross-section of the air conditioner according to an embodiment of the present disclosure.

On the other hand, 'upper' and 'upward' may be a direction facing the ceiling of an indoor space in which an air conditioner 1 is installed. 'Lower' and 'downward' may be a direction facing the ground of the indoor space in which the air conditioner 1 is installed.

'Front' may be a direction toward a center within a housing 10 of the air conditioner 1. In other words, 'front' may be a direction toward the center of a circle. 'Front' may be a direction toward a blower fan 50 disposed approximately at the center of the housing 10. 'Rear' may be a radial direction of the circle. 'Rear' may be a direction from the center of the blower fan 50 toward the outside of the blower fan 50.

Referring to FIGS. 1 to 3, the air conditioner 1 may be mounted on the ceiling. At least a portion of the air conditioner 1 may be embedded in the ceiling. The air conditioner 1 may be an indoor unit 1.

The air conditioner 1 may include the housing 10. The housing 10 may form an exterior of the air conditioner 1. The housing 10 may include a cabinet 17 and a lower panel 11 coupled to the cabinet 17.

The cabinet 17 may be provided in an approximately square shape. The cabinet 17 may be buried in the ceiling and arranged to be open downwardly.

The cabinet 17 may include a base 18 mounted horizontally on the ceiling and an outer wall 19 extending downwardly from an outer side of the base 18. The cabinet 17 may be formed to be open downwardly by a configuration of an opening formed by the base 18 and the outer wall 19.

The lower panel 11 may be positioned on a lower side of the cabinet 17. The lower panel 11 may form a lower exterior of the air conditioner 1. The lower panel 11 may have an approximately square shape.

The lower panel 11 may include a lower plate 12. The lower plate 12 may include an inlet 15 provided to allow indoor air to be drawn into the air conditioner 1 and an outlet 16 provided to allow air inside the air conditioner 1 to be discharged into the indoor space.

The inlet 15 may be formed approximately at the center of the lower panel 11. The inlet may have a circular shape.

The outlets 16 may be formed in four and arranged on sides facing each other, and each of which may have a shape extending in a X direction or a Y direction. The outlets 16 may be formed in four as shown in the drawings, but are not limited thereto.

The air conditioner 1 may include a separate cover member (not shown) at a lower side. The cover member (not shown) may have a square shape or a circular shape depending on how it is installed on the ceiling.

The air conditioner 1 may include an inlet grille 25 installed at the inlet 15 of the lower panel 11 to allow indoor air to be drawn into the air conditioner 1. The inlet grille 25 may filter out foreign substances in the air entering the interior of the cabinet 17 through the inlet 15.

The air conditioner 1 may include the blower fan 50 that flows air. A fan motor 55 provided to drive the blower fan 50 may be disposed on an upper portion of the blower fan 50.

The blower fan 50 may include a fan hub 54 and a fan blade 51. The fan hub 54 may be connected to a rotation shaft 56 of the fan motor 55 and may be rotated by the power of the fan motor 55. The fan blade 51 may be radially connected to the fan hub 54 and may rotate together with the rotation of the fan hub 54 to transfer air.

The fan blade 51 may include an outer circumferential surface 51a and an inner circumferential surface 51b. The inner circumferential surface 51b may be disposed toward the center of the blower fan 50. The outer circumferential surface 51a may be disposed toward a radial direction of the blower fan 50.

The air conditioner 1 may include a heat exchanger 60 configured to heat exchange air discharged from the blower fan 50. Based on the air conditioner 1 being in a cooling mode, the air discharged from the blower fan 50 may be cooled by the heat exchanger 60. Similarly, based on the air conditioner 1 being in a heating mode, the air discharged from the blower fan 50 may be heated by the heat exchanger 60.

A pipe portion 61 may be formed at a corner of the heat exchanger 60. The refrigerant in the heat exchanger 60 may be discharged through the pipe portion 61.

The heat exchanger 60 may be arranged radially away from the blower fan 50. The heat exchanger 60 may be formed by bending to correspond to the shape of the inner circumferential surface of the cabinet 17. The heat exchanger 60 may have an approximately square shape. The heat exchanger 60 may be arranged to surround the blower fan 50.

The air conditioner 1 may include a drain pan 30 that stores condensed water generated by the heat exchanger 60. The drain pan 30 may be disposed between the lower panel 11 and the cabinet 17. The drain pan 30 may be positioned at a lower portion of the heat exchanger 60. The drain pan 30 may support the heat exchanger 60 and store condensed water generated by the heat exchanger 60.

At approximately the center of the drain pan 30, a drain pan opening 33 may be formed through which air is drawn into the interior of the cabinet 17. The inlet grille 25 may be coupled to the drain pan opening 33.

The air conditioner 1 may include a sterilization device 100. The sterilization device 100 may sterilize the interior of the air conditioner 1.

The blower fan 50 may flow the air heat exchanged by the heat exchanger 60 to the outlets 16. At this time, moisture may condense on the fan blade 51 due to contact with the heat exchanged air, and bacteria may multiply. As a result, a foul odor may be generated by the bacteria, and the bacteria may be discharged into the indoor air.

At this time, the sterilization device 100 may sterilize the blower fan 50 by irradiating light toward the blower fan 50. In other words, the sterilization device 100 may sterilize the interior of the housing 10.

The sterilization device 100 may be disposed within the housing 10. Referring to FIG. 3, the sterilization device 100 may be disposed on an upper portion of the drain pan 30. The sterilization device 100 may be arranged to irradiate light toward the blower fan 50. The sterilization device 100 may be mounted on a radially outer side of the blower fan 50. The sterilization device 100 may be disposed adjacent to the outer circumferential surface 51a of the fan blade 51. The sterilization device 100 may be disposed between the blower fan 50 and the heat exchanger 60 to irradiate light toward the blower fan 50.

The sterilization device 100 may emit ultraviolet rays to the blower fan 50 when the blower fan 50 is operated. In other words, in response to an irradiation mode being operated in the air conditioner 1, the blower fan 50 may be rotated by the fan motor 55. At this time, the sterilization device 100 may irradiate light toward the fan blade 51, and the light irradiated by the sterilization device 100 when the blower fan 50 rotates may be uniformly irradiated to the entire area of the fan blade 51.

The sterilization device 100 may include a case 200 that accommodates a light-emitting module 110 and a holder 300 that supports the case 200. The case 200 may be disposed on an upper portion of the holder 300. The holder 300 may be disposed to face the lower panel 11.

The light-emitting module 110 may be disposed to be horizontally spaced apart from the blower fan 50 by about 19 to 22 mm. However, the present disclosure is not limited thereto. A spacing distance between the light-emitting module 110 and the blower fan 50 may be varied in consideration of variables, such as sterilization performance, noise, and the like.

Specifically, the closer the light-emitting module 110 is disposed to the blower fan 50, the better the sterilization performance for the blower fan 50 may be. Whereas, the closer the light-emitting module 110 is disposed to the blower fan 50, the more noise may be generated. In other words, the spacing distance between the light-emitting module 110 and the blower fan 50 may be inversely correlated with the sterilization performance and proportionally correlated with noise.

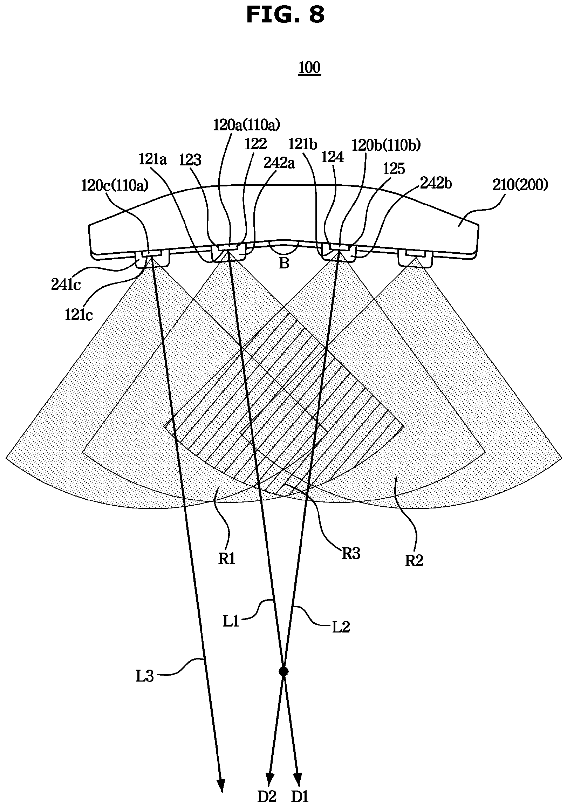

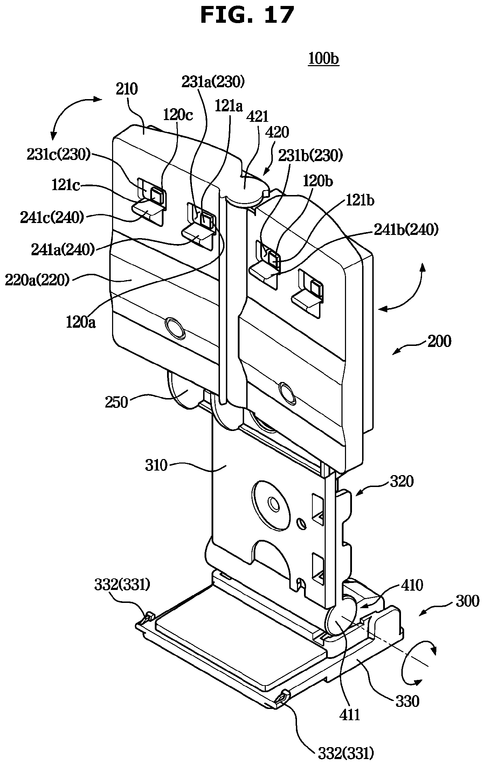

Accordingly, it is necessary to set an appropriate distance between the light-emitting module 110 and the blower fan 50, wherein the angle of the light emitted by the light-emitting module 110 may be adjusted to compensate for the sterilization performance that decreases as the distance between the light-emitting module 110 and the blower fan 50 increases. For example, referring to FIG. 8, the light may be overlapped depending on the angle of arrangement of a light-emitting diode (LED) 120. In addition, referring to FIG. 17, the overlapping range of light may be adjusted by adjusting the arrangement angle of the LED 120.

As the overlapping range of light emitted toward the blower fan 50 increases, the sterilization performance for the blower fan 50 may be improved.

The air conditioner 1 may include a sterilization coupling portion 70 provided to couple the sterilization device 100. The sterilization coupling portion 70 may be disposed within the housing 10. The sterilization coupling portion 70 may be configured to secure the sterilization device 100 within the housing 10.

The sterilization coupling portion 70 may be arranged in the drain pan 30. The sterilization coupling portion 70 may include a drain pan coupling portion 31. The sterilization device 100 may be mounted on the drain pan 30. The holder 300 may be coupled to the drain pan coupling portion 31.

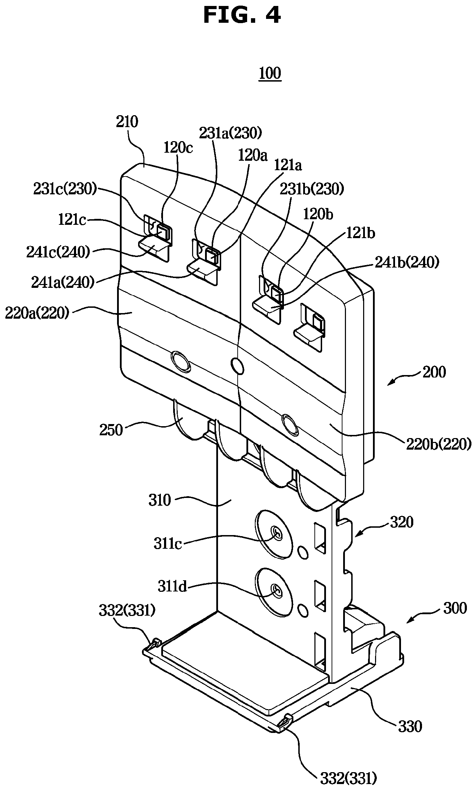

FIG. 4 is a front perspective view showing the sterilization device according to an embodiment of the present disclosure.

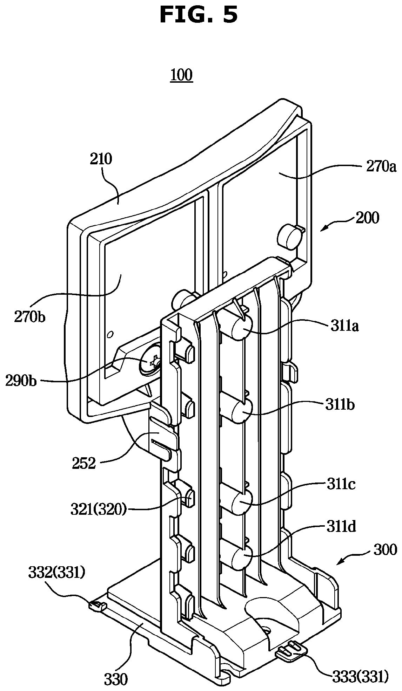

FIG. 5 is a rear perspective view showing the sterilization device according to an embodiment of the present disclosure.

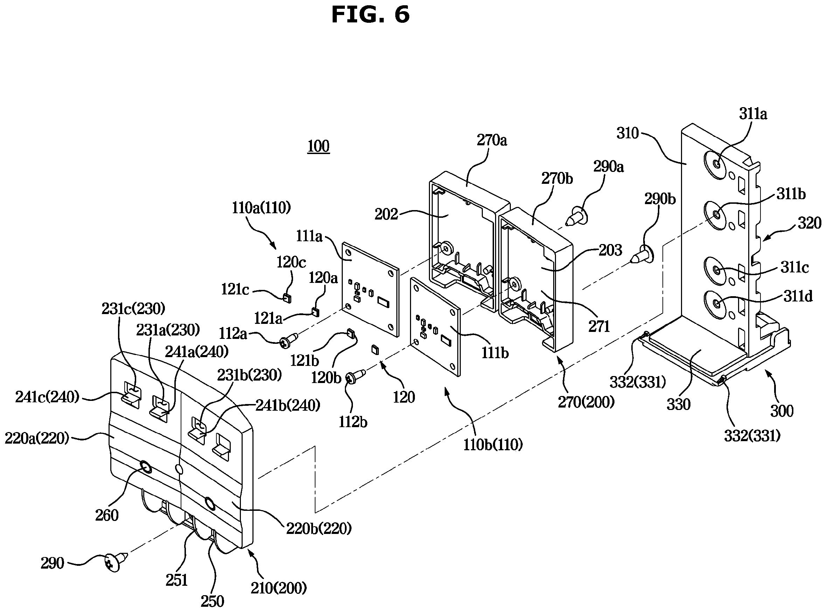

FIG. 6 is an exploded front perspective view showing a configuration of the sterilization device according to an embodiment of the present disclosure.

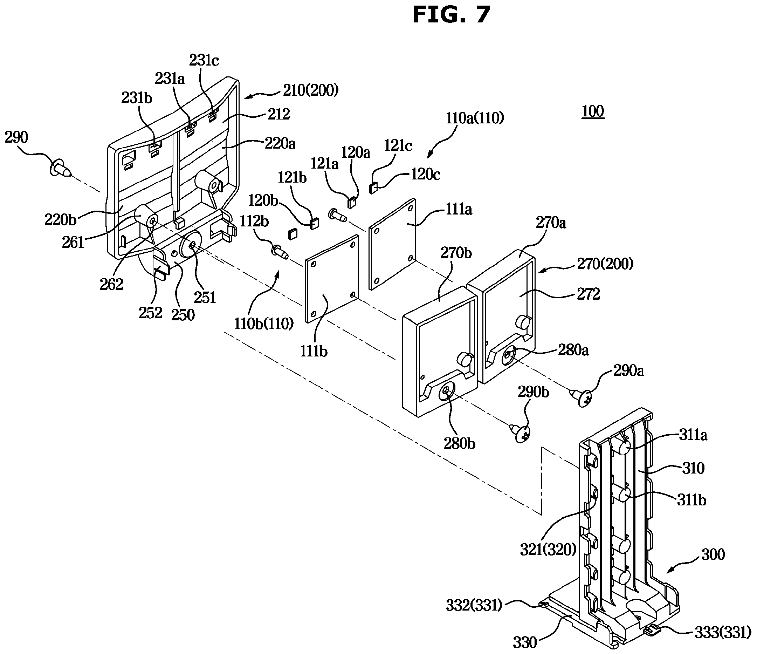

FIG. 7 is an exploded rear perspective view showing the configuration of the sterilization device according to an embodiment of the present disclosure.

The sterilization device 100 may include the light-emitting module 110. The light-emitting module 110 may include the LED 120 and a substrate 111 on which the LED 120 is mounted. The light-emitting module 110 may receive power and emit light. The light-emitting module 110 may emit ultraviolet rays.

The light-emitting module 110 may include a first light-emitting module 110a and a second light-emitting module 110b. The first light-emitting module 110a and/or the second light-emitting module 110b may each include one substrate 111 and two LEDs 120. However, the present disclosure is not limited thereto, and the first light-emitting module 110a and/or the second light-emitting module 110b may each include one LED 120, or may each include three or more LEDs 120.

The substrate 111 may be provided with a port to which power is supplied, and a connector for supplying power to the port may be connected. The substrate 111 may be configured to apply power to the LED 120 to cause the LED 120 to emit light.

The LED 120 may include a first LED 120a and a second LED 120b spaced apart from the first LED 120a.

The LED 120 may include an irradiation surface 121 that irradiates light toward the outside. The irradiation surface 121 may be disposed on the opposite side of the surface in which the LED 120 is in contact with the substrate 111.

The sterilization device 100 may include the case 200 that accommodates the light-emitting module 110. The case 200 may form an accommodation space 201 therein.

The case 200 may include a front case 210 and a rear case 270. The front case 210 and the rear case 270 may be coupled together to form the accommodation space 201. However, the present disclosure is not limited thereto, and the case 200 may be formed integrally.

The front case 210 may be disposed on a front side of the rear case 270. The front case 210 may be arranged in front of the light-emitting module 110. The front case 210 may be disposed adjacent to the blower fan 50.

The front case 210 may include a cover portion 220. The cover portion 220 may prevent the substrate 111 from being exposed to the outside. The cover portion 220 may have a plate shape. The cover portion 220 may be disposed on a front side of the substrate 111.

The cover portion 220 may include a first cover portion 220a that covers the first light-emitting module 110a and a second cover portion 220b that covers the second light-emitting module 110b.

The front case 210 may include an irradiation portion 230. The irradiation portion 230 may be provided to irradiate light generated from the LED 120 toward the outside. The irradiation portion 230 may be arranged to irradiate light toward the blower fan 50. The irradiation portion 230 may be arranged to irradiate light generated from the LED 120 toward the outside of the sterilization device 100. The irradiation portion 230 may be arranged such that the irradiation surface 121 of the LED 120 is exposed to the outside.

The irradiation portion 230 may include a first irradiation portion provided to allow light generated from the first light-emitting module 110a to be emitted to the outside, and a second irradiation portion provided to allow light generated from the second light-emitting module 110b to be emitted to the outside.

The irradiation portion 230 may include an irradiation hole 231. The irradiation hole 231 may be formed to penetrate the front case 210. The irradiation hole 231 may be formed corresponding to a position of the LED 120. The irradiation hole 231 may be formed on a front side of the LED 120.

The irradiation hole 231 may include a first irradiation hole 231a corresponding to the first LED 120a and a second irradiation hole 231b corresponding to the second LED 120b.

However, the present disclosure is not limited thereto, and the irradiation portion 230 may include quartz glass or the like provided to allow light to pass therethrough.

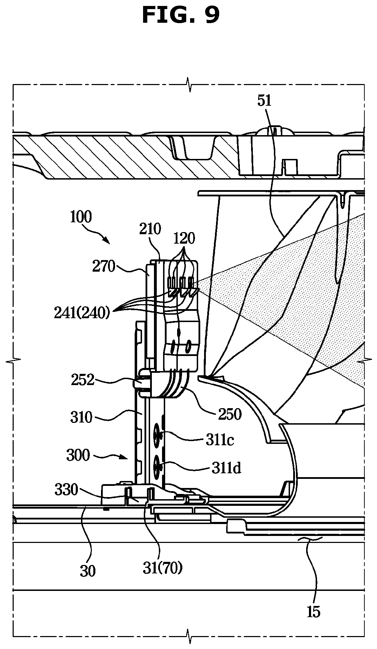

The front case 210 may include a blocking portion 240. The blocking portion 240 may regulate the range in which light generated from the LED 120 is irradiated. The blocking portion 240 may prevent light from being exposed to the outside of the housing 10. The blocking portion 240 may prevent light from penetrating the inlet 15 and/or the outlets 16 of the lower panel 11 and being exposed to the outside. The blocking portion 240 may adjust the angle at which the light is irradiated. This will be described later with reference to FIG. 9.

The drawings illustrate an example in which the blocking portion 240 is formed on the front case 210, but the present disclosure is not limited thereto. The blocking portion 240 may be formed on a configuration other than the front case 210.

The blocking portion 240 may include a first blocking portion that regulates the range in which light generated from the first light-emitting module 110a is irradiated and a second blocking portion that regulates the range in which light generated from the second light-emitting module 110b is irradiated.

The blocking portion 240 may include a blocking protrusion 241. The blocking protrusion 241 may be formed to protrude from a front surface 211 of the front case. The blocking protrusion 241 may protrude forwardly. The blocking protrusion 241 may protrude toward the blower fan 50.

The blocking protrusion 241 may include a plate shape. The blocking protrusion 241 may include a rib shape. The blocking protrusion 241 may be formed integrally with the front case 210.

The blocking protrusion 241 may be disposed on a lower portion of the LED 120. The blocking protrusion 241 may be disposed on a bottom of the irradiation surface 121 to block download light of the light emitted from the irradiation surface 121. The blocking protrusion 241 may be provided at a bottom of the irradiation hole 231.

The blocking protrusion 241 may extend along the length of the LED 120.

The blocking protrusion 241 may include a first blocking protrusion 241a provided to prevent light emitted by the first LED 120a from being directed downwardly, and a second blocking protrusion 241b provided to prevent light emitted by the second LED 120b from being directed downwardly.

The front case 210 may include an extension 250. The extension 250 may extend downwardly from the cover portion 220. The extension 250 may include a case coupling hole 251. The front case 210 may be coupled to the holder 300 by the case coupling hole 251.

The rear case 270 may be disposed on a rear side of the front case 210. The rear case 270 may be disposed on a rear side of the light-emitting module 110. The rear case 270 may be disposed adjacent to the heat exchanger 60.

The rear case 270 may secure the light-emitting module 110. The substrate 111 may be secured to a front surface 271 of the rear case.

The rear case 270 may include a first rear case 270 that accommodates the first light-emitting module 110a and a second rear case 270 that accommodates the second light-emitting module 110b. Although the first rear case 270 and the second rear case 270 are shown in the drawings to be formed separately as an example, but the present disclosure is not limited thereto. For example, the first rear case 270 and the second rear case 270 may be formed integrally. Alternatively, the rear case 270 may be formed as a single configuration that accommodates both the first light-emitting module 110a and the second light-emitting module 110b.

The case 200 may be fastened by a front case fastening portion 260 in the front case 210 and a rear case fastening hole 280 in the rear case 270.

The front case fastening portion 260 may include a front case fastening protrusion 261. The front case fastening protrusion 261 may be formed on a rear surface 212 of the front case. The front case fastening protrusion 261 may protrude toward the rear case 270.

The front case fastening protrusion 261 may include a front case fastening groove 262. The front case fastening groove 262 may be formed by a recess at approximately the center of the front case fastening protrusion 261. The front case fastening groove 262 may be formed by concave recess toward the front.

The rear case fastening portion may include the rear case fastening hole 280. The rear case fastening hole 280 may be formed to penetrate the rear case 270. The rear case fastening hole 280 may be disposed to correspond to a position of the front case fastening groove 262.

A case fastening screw 290 may penetrate the rear case fastening hole 280 and be inserted into the front case fastening groove 262, thereby fastening the front case 210 to the rear case 270.

The sterilization device 100 may include the holder 300. The holder 300 may support the case 200. The holder 300 may be arranged to securely hold the sterilization device 100 within the housing 10. The holder 300 may be arranged to be coupled to the drain pan 30, so that the light-emitting module 110 within the case 200 may be disposed to be spaced apart from the drain pan 30.

The holder 300 may include a first frame 310 coupled to the case 200 and a second frame 330 connected to the first frame 310.

The first frame 310 may be disposed on an upper portion of the second frame 330. The first frame 310 may extend vertically. The second frame 330 may extend horizontally.

The first frame 310 may include a holder coupling hole 311 to allow the case 200 to be coupled thereto. The holder coupling hole 311 may be formed to correspond to the case coupling hole 251 of the front case 210.

The holder coupling hole 311 may include a first hole 311a and a second hole 311b spaced apart from the first hole 311a. The second hole 311b may be positioned lower than the first hole 311a. The holder coupling holes 311 are provided in a plurality, and thus the front case 210 may be coupled to different heights of the first frame 310. As a result, the height at which the light-emitting module 110 is disposed may be adjusted, and a user may adjust the height of the light-emitting module 110 in response to the size of the blower fan 50.

The first frame 310 may include a cable receiving portion 320. The cable receiving portion 320 may receive a cable connected to the light-emitting module 110. The cable receiving portion 320 may include a hook 321. The cable may be inserted into the hook 321 and secured to a rear surface of the first frame 310. The hooks 321 may be provided in a plurality.

The second frame 330 may be coupled to the first frame 310. However, the present disclosure is not limited thereto, and the first frame 310 and the second frame 330 may be integrally formed.

The second frame 330 may include a second frame coupling portion 331 coupled to the drain pan 30. The second frame coupling portion 331 may include a second frame coupling hook 321. The second frame coupling hook 321 may be disposed on a front side of the second frame 330. The second frame coupling hook 321 may be provided in a plurality.

The second frame coupling portion 331 may include a second frame coupling protrusion 333. The second frame coupling protrusion 333 may be disposed on the rear side of the second frame 330. The second frame coupling protrusion 333 may be formed to protrude rearwardly. The second frame coupling protrusion 333 may be formed at approximately the center of a rear end of the second frame 330.

FIG. 8 is a top view showing light irradiated from the sterilization device according to an embodiment of the present disclosure.

The LED 120 may include the irradiation surface 121. Light may be irradiated from the irradiation surface 121. The irradiation surface 121 may be formed on the opposite side of the rear surface of the substrate 111 where the LED 120 contacts the substrate 111. The irradiation surface 121 may be the front surface of the LED 120. The irradiation surface 121 may be formed as a plane.

The LED 120 may be disposed parallel to the substrate 111. The irradiation surface 121 may be disposed on the front side of the substrate 111, parallel to the substrate 111.

The LED 120 may include the first LED 120a and the second LED 120b spaced apart from the first LED 120a. A distance from the center of the first LED 120a to the center of the second LED 120b may be about 15 to 21 mm. However, the present disclosure is not limited thereto.

The first LED 120a may include a first irradiation surface 121a that irradiates light, and the second LED 120b may include a second irradiation surface 121b that irradiates light.

A first extension line L1 may be defined as an imaginary line extending orthogonally to the first irradiation surface 121a of the first LED 120a. The first extension line L1 may extend orthogonally to a first substrate 111a. The first extension line L1 may extend from the first irradiation surface 121a in a front-to-back direction. An angle formed by the first extension line L1 and the first irradiation surface 121a may be 90 degrees.

A second extension line L2 may be defined as an imaginary line extending orthogonally to the second irradiation surface 121b of the second LED 120b. The above description of the first extension line L1, the first LED 120a, the first irradiation surface 121a, and the first substrate 111a may be replaced by a description of the second extension line L2, the second LED 120b, the second irradiation surface 121b, and a second substrate 111b.

The first extension line L1 may be arranged to intersect the second extension line L2. The first extension line L1 and the second extension line L2 may meet at a point A. The point A at which the first extension line L1 and the second extension line L2 intersect may be arranged to form an acute angle between 0 and 90 degrees. The first extension line L1 and the second extension line L2 may be arranged such that they are not parallel to each other.

In other words, the first irradiation surface 121a and the second irradiation surface 121b may be arranged to be inclined with respect to each other. The first irradiation surface 121a and the second irradiation surface 121b may not be arranged along one direction. The first irradiation surface 121a and the second irradiation surface 121b may be arranged such that they are not to be parallel to each other.

The first LED 120a may be arranged such that the first irradiation surface 121a faces a first direction D1. The first direction D1 may be a direction along the first extension line L1.

The second LED 120b may be arranged such that the second irradiation surface 121b faces a second direction D2. The second direction D2 may be a different direction from the first direction D1. The second direction D2 may be a direction along the second extension line L2.

The first irradiation surface 121a may include a first end 122 disposed adjacent to the second irradiation surface 121b and a second end 123 disposed opposite the first end 122. The first end 122 may be disposed further back than the second end 123. The first irradiation surface 121a may be arranged to be inclined toward the rear as it approaches the second irradiation surface 121b.

The second irradiation surface 121b may include a first end 124 disposed adjacent to the first irradiation surface 121a and a second end 125 disposed opposite the first end 124. The first end 124 may be disposed to face the first end 122. The first end 124 may be disposed further back than the second end 125. The second irradiation surface 121b may be arranged to be inclined rearwardly as it approaches the first irradiation surface 121a.

The first irradiation surface 121a and the second irradiation surface 121b may be disposed to be inclined toward the rear as they approach each other. The first irradiation surface 121a and the second irradiation surface 121b may be arranged to be inclined toward the rear as they approach a center between the first LED 120a and the second LED 120b.

The first LED 120a may be arranged to be inclined forwardly as it is further away from the second LED 120b. The second LED 120b may be arranged to be inclined toward the front as it is further away from the first LED 120a.

The case 200 may include a first surface 202 on which the first LED 120a is seated and a second surface 203 on which the second LED 120b is seated. The first surface 202 and/or the second surface 203 may be formed on the front surface 271 of the rear case (see FIG. 6). The first surface 202 may be arranged to be perpendicular to the first extension line L1. The second surface 203 may be arranged to be perpendicular to the second extension line L2.

The light irradiated from the irradiation surface 121 may form a certain irradiation range. The light irradiated from the first irradiation surface 121a may be irradiated in a first irradiation range R1, and the light irradiated from the second irradiation surface 121b may be irradiated in a second irradiation range R2. The first irradiation range R1 and the second irradiation range R2 may form an overlapping third irradiation range R3. The sterilization performance of the third irradiation range R3 may be improved because the light is overlapped. In other words, the sterilization performance may be improved by concentrating the irradiation of light.

In this case, the first irradiation surface 121a and the second irradiation surface 121b may be arranged at an angle, so that the area of the third irradiation range R3 may be enlarged. In other words, since an arrangement angle B formed between the first irradiation surface 121a and the second irradiation surface 121b is formed to be less than 180 degrees, so that the area where the first irradiation range R1 and the second irradiation range R2 overlap may be enlarged, thereby improving the sterilization performance can be improved.

The arrangement angle B between the first irradiation surface 121a and the second irradiation surface 121b may be between 150 and 175 degrees. However, the present disclosure is not limited thereto.

The first irradiation surface 121a may be arranged to be inclined toward the blower fan 50 as it is further away from the second irradiation surface 121b, so that the area of the third irradiation range R3 may be increased. Accordingly, the sterilization performance may be improved by focusing the irradiation of light on the blower fan 50.

The LED 120 may include a third LED 120c spaced apart from the first LED 120a and the second LED 120b. The third LED 120c may include a third irradiation surface 121c that irradiates light.

The LED 120 may be provided in a plurality, and thus the area of the irradiation range where the light overlaps may be increased, thereby improving the sterilization function of the sterilization device 100.

The third LED 120c may be mounted on the first substrate 111a on which the first LED 120a is mounted.

A third extension line L3 may be defined as an imaginary line extending orthogonally to the third irradiation surface 121c of the third LED 120c. The above description of the first extension line L1, the first LED 120a, and the first irradiation surface 121a may be replaced by a description of the third extension line L3, the third LED 120c, and the third irradiation surface 121c.

The third extension line L3 may be arranged to be parallel to the first extension line L1. The third extension line L3 may be arranged to intersect the second extension line L2 at one point.

FIG. 9 is a view showing the sterilization device according to an embodiment of the present disclosure irradiating light toward the blower fan.

The blocking protrusion 241 may prevent light irradiated from the LED 120 from leaking out of the air conditioner 1. The blocking protrusion 241 may prevent light from being directed to the outlets 16 and/or the inlet 15. The blocking protrusion 241 may adjust the angle of irradiation of light directed downward. The blocking protrusion 241 may be disposed between the LED 120 and the lower panel 11.

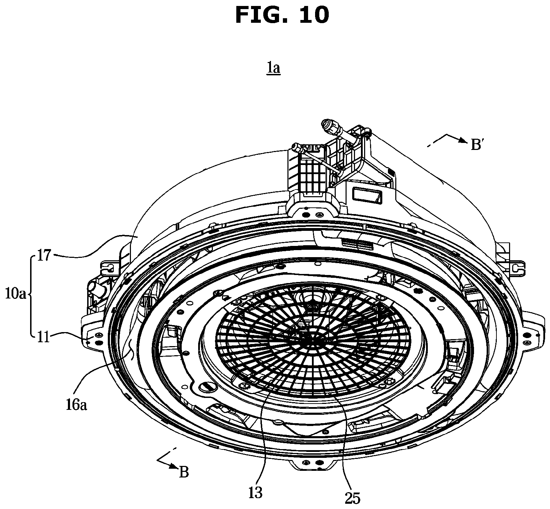

FIG. 10 is a perspective view showing an air conditioner according to an embodiment of the present disclosure.

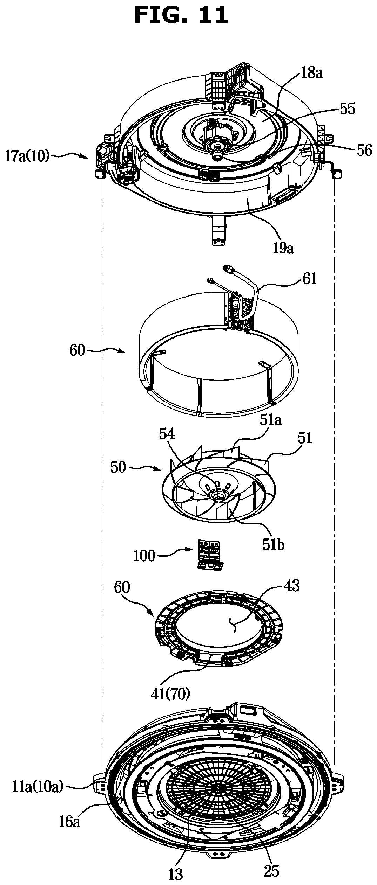

FIG. 11 is an exploded perspective view showing a configuration of the air conditioner according to an embodiment of the present disclosure.

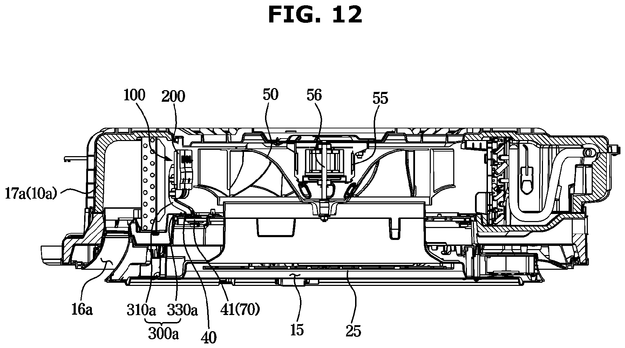

FIG. 12 is a cross-sectional view showing a cross-section of the air conditioner according to an embodiment of the present disclosure.

A description of the configuration and content that is redundant to FIGS. 1 to 9 described above will be omitted.

A housing 10a of an air conditioner 1a may include a cabinet 17a and a lower panel 11a. The cabinet 17a may be provided in an approximately circular shape. The lower panel 11a may be provided in an approximately circular shape.

The lower panel 11a may include an outlet 16a provided to discharge air inside the air conditioner 1a into a room. The outlet 16a may extend along a circumferential direction.

The air conditioner 1a may include a bell mouth 40. The bell mouth 40 may guide air drawn into the inlet 15 toward the blower fan 50. The bell mouth 40 may be disposed at a lower portion of the blower fan 50. The bell mouth 40 may extend along the circumferential direction.

The sterilization coupling portion 70 may be provided on the bell mouth 40. The sterilization coupling portion 70 may include a bell mouth coupling portion 41. The sterilization device 100 may be mounted on the bell mouth 40. The holder 300 may be coupled to the bell mouth coupling portion 41.

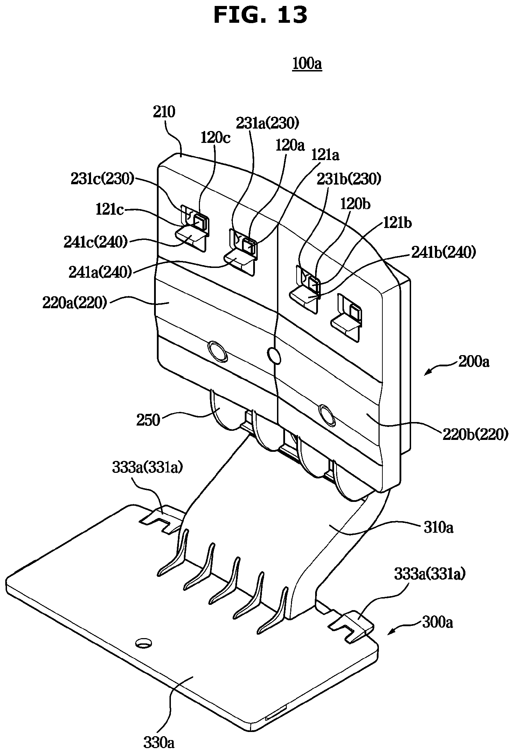

FIG. 13 is a front perspective view showing a sterilization device according to an embodiment of the present disclosure.

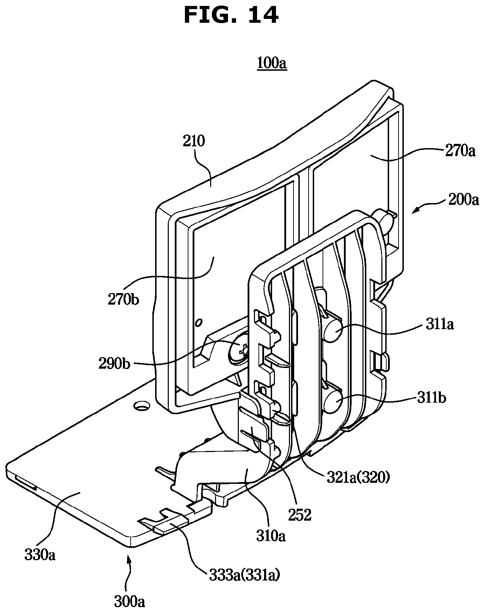

FIG. 14 is a rear perspective view showing the sterilization device according to an embodiment of the present disclosure.

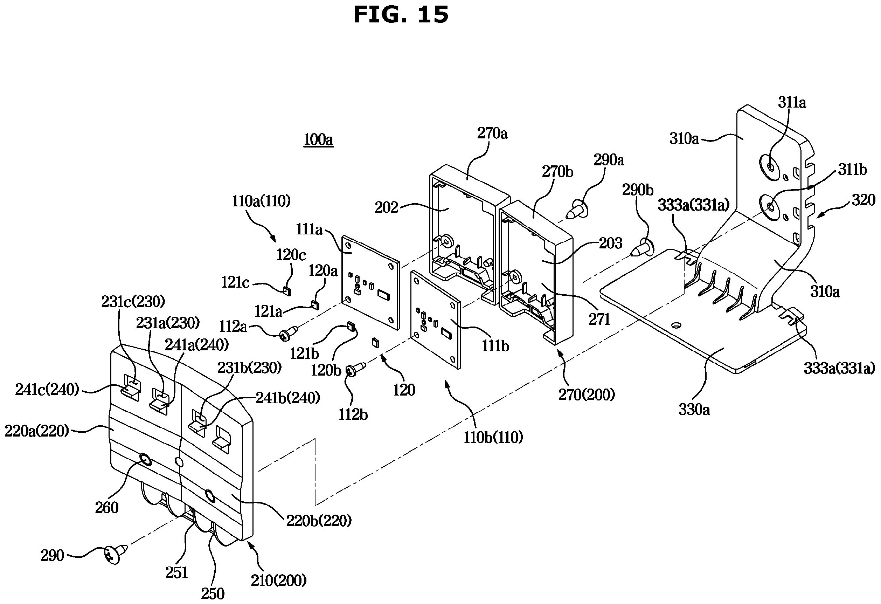

FIG. 15 is an exploded front perspective view showing a configuration of the sterilization device according to an embodiment of the present disclosure.

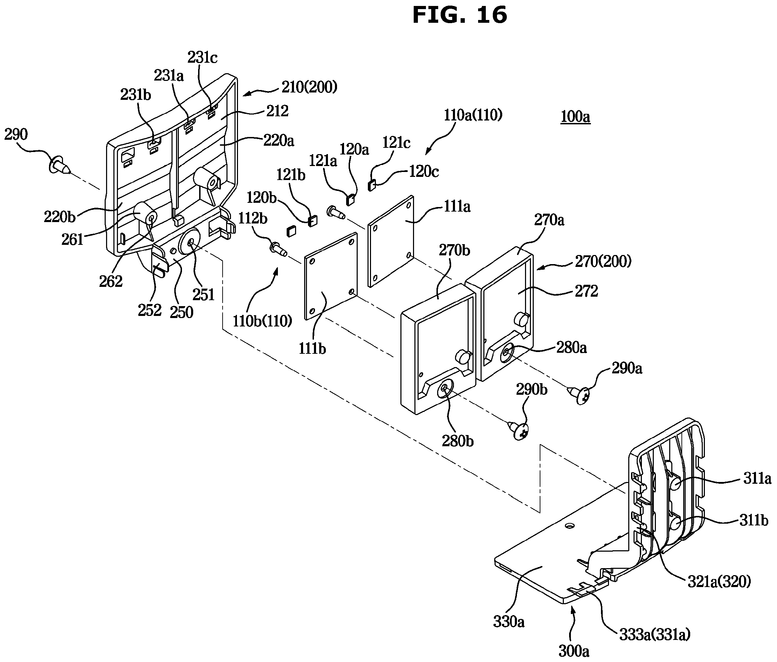

FIG. 16 is an exploded rear perspective view showing the configuration of the sterilization device according to an embodiment of the present disclosure.

A description of the configuration and content that is redundant to FIGS. 1 to 9 described above will be omitted.

A holder 300a may include a first frame 310a and a second frame 330a. The second frame 330a may extend from the first frame 310a.

The second frame 330a may include a second frame coupling portion 331a corresponding to the bell mouth coupling portion 41. The second frame coupling portion 331a may include a second frame coupling protrusion 333a. The second frame coupling protrusion 333a may be provided on a rear side of the second frame 330a. The second frame coupling protrusion 333a may be formed to protrude rearwardly from a rear end of the second frame 330a. The second frame coupling protrusion 333a may be provided in a plurality.

FIG. 17 is a front perspective view showing a sterilization device according to an embodiment of the present disclosure.

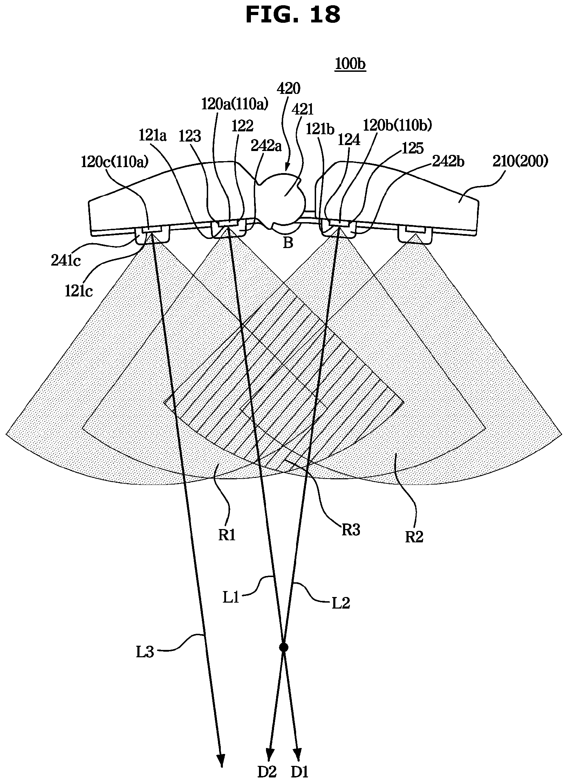

FIG. 18 is a top view showing light irradiated from the sterilization device according to an embodiment of the present disclosure.

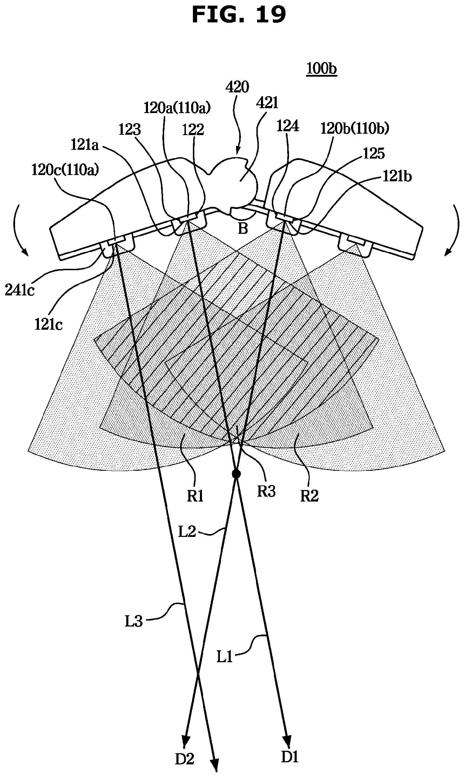

FIG. 19 is a top view of the sterilization device of FIG. 18 being rotated.

A description of the configuration and content that is redundant to FIGS. 1 to 16 described above will be omitted.

The sterilization device 100 may include a first hinge portion 410. The first hinge portion 410 may be provided to allow the first frame 310 to rotate relative to the second frame 330. The first hinge portion 410 may adjust the arrangement angle of the first frame 310 relative to the second frame 330. The first hinge portion 410 may be disposed between the first frame 310 and the second frame 330.

A spacing distance between the first frame 310 and the blower fan 50 may be adjusted by the first hinge portion 410. As a result, the distance between the light-emitting module 110 and the blower fan 50 may be adjusted, thereby adjusting the distance as needed.

The first hinge portion 410 may include a holder hinge 411. The holder hinge 411 may be formed on a lower portion of the first frame 310. The holder hinge 411 may connect the first frame 310 and the second frame 330.

The sterilization device 100 may include a second hinge portion 420. The second hinge portion 420 may be provided to allow the second light-emitting module 110b to rotate relative to the first light-emitting module 110a. The second hinge portion 420 may be provided to allow the second LED 120b to rotate relative to the first LED 120a. The second hinge portion 420 may adjust the arrangement angle B between the first LED 120a and the second LED 120b.

The second hinge portion 420 may include a case hinge 421. The case hinge 421 may be provided in the case 200. The case hinge 421 may be formed in the front case 210. The case hinge 421 may be a hinge provided between the first cover portion 220a and the second cover portion 220b.

Referring to FIGS. 18 and 19, the arrangement angle B between the first LED 120a and the second LED 120b may be adjusted by the case hinge 421. The smaller the arrangement angle B becomes, the more the irradiation range where the light overlaps increases, thereby improving the sterilization performance. On the other hand, the smaller the arrangement angle B becomes, the more the resistance value to air flow increases, resulting in noise. Accordingly, the case hinge 421 may be adjusted to provide an appropriate range of the arrangement angle B.

As described above in FIGS. 1 to 3, the sterilization performance and the noise level may be adjusted according to the spacing distance between the light-emitting module 110 and the blower fan 50. Accordingly, the noise may be reduced by adjusting the spacing distance between the light-emitting module 110 and the blower fan 50 via the first hinge portion 410, and the sterilization performance may be improved by adjusting the overlapping range of light by changing the arrangement angle B between the LEDs 120 via the second hinge portion 420.

For example, when the light-emitting module 110 is spaced farther away from the blower fan 50 by adjusting the first hinge portion 410, the second hinge portion 420 may be adjusted to decrease the arrangement angle B, thereby increasing the irradiation range where the light overlaps to maintain the sterilization performance.

Conversely, when the light-emitting module 110 is disposed adjacent to the blower fan 50 by adjusting the first hinge portion 410, the arrangement angle B may be increased by adjusting the second hinge portion 420, thereby reducing the irradiation range where the light overlaps.

On the other hand, the spacing distance between the blower fan 50 and the light-emitting module 110 may be about 19 to 22 mm. The arrangement angle B between the first LED 120a and the second LED 120b may be between 150 to 175 degrees.

The embodiment of FIGS. 10 to 16 may be combined with the embodiment of FIGS. 8 to 9.

The embodiment of FIGS. 17 to 19 may be combined with the embodiment of FIGS. 10 to 16.

While the present disclosure has been particularly described with reference to exemplary embodiments, it should be understood by those of skilled in the art that various changes in form and details may be made without departing from the spirit and scope of the present disclosure.

## Claims

1. An air conditioner 1 or 1a, comprising:
a housing 10 having an inlet 15 and an outlet 16;
a heat exchanger 60 disposed within the housing 10 to heat exchange with air drawn in through the inlet 15;
a blower fan 50 configured to flow heat exchanged air to allow the air heat exchanged by the heat exchanger 60 to be discharged through the outlet 16; and
a sterilization device 100 configured to irradiate light toward the blower fan 50;
wherein the sterilization device 100, 100a or 100b comprises:
a first light-emitting diode (LED) 120a including a first irradiation surface 121a, and
a second LED 120b including a second irradiation surface 121b,
wherein the first LED 120a is arranged such that the first irradiation surface 121a faces a first direction D1, and the second LED 120b is arranged such that the second irradiation surface 121b faces a second direction D2 different from the first direction D1.

2. The air conditioner 1 or 1a of claim 1, wherein the sterilization device 100, 100a or 100b further includes a third LED 120c spaced apart from the first LED 120a and including a third irradiation surface 121c, and
the third LED 120c is arranged such that the third irradiation surface 121c faces the first direction D1.

3. The air conditioner 1 or 1a of claim 1, wherein the sterilization device 100, 100a or 100b further includes a blocking protrusion 241 configured to prevent light from being exposed to an outside of the housing 10.

4. The air conditioner 1 or 1a of claim 3, wherein the blocking protrusion 241 comprises:
a first blocking protrusion 241a extending toward the first direction D1, and
a second blocking protrusion 241b extending toward the second direction D2.

5. The air conditioner 1 or 1a of claim 4, whereinthe housing 10 further includes a lower panel 11 in which the inlet 15 and the outlet 16 are formed,
the first blocking protrusion 241a is disposed at a lower end of the first irradiation surface 121a to prevent light irradiated from the first LED 120a from penetrating the lower panel 11, and
the second blocking protrusion 241b is disposed at a lower end of the second irradiation surface 121b to prevent light irradiated from the second LED 120b from penetrating the lower panel 11.

6. The air conditioner 1 or 1a of claim 1, wherein the heat exchanger 60 is arranged to surround the blower fan 50, and
the sterilization device 100 is disposed between the heat exchanger 60 and the blower fan 50.

7. The air conditioner 1 or 1a of claim 6, wherein the first LED 120a is arranged to be inclined toward the blower fan 50 as the first LED 120a is further away from the second LED 120b.

8. The air conditioner 1 or 1a of claim 1, wherein the sterilization device 100, 100a or 100b further comprises:
a case 200 or 200a configured to accommodate the first LED 120a and the second LED 120b, and
a holder 300 or 300a configured to support the case 200 or 200a.

9. The air conditioner 1 or 1a of claim 8, wherein the case 200a further includes a case hinge 421 configured to allow the second LED 120b to rotate relative to the first LED 120a.

10. The air conditioner 1 or 1a of claim 8, further comprising a sterilization coupling portion 70 disposed within the housing 10 and coupled to the holder 300 or 300a in order to support the sterilization device 100, 100a or 100b,
wherein the holder 300 or 300a comprises:
a first frame 310 or 310a couplable to the case 200, and
a second frame 330 or 330a connected to the first frame 310 or 310a and couplable to the sterilization coupling portion 70.

11. The air conditioner 1 or 1a of claim 10, further comprising a drain pan 30 arranged on a lower portion of the heat exchanger 60 and storing condensed water,
wherein the sterilization coupling portion 70 is arranged at the drain pan 30.

12. The air conditioner 1 or 1a of claim 10, further comprising a bell mouth 40 configured to guide air drawn in through the inlet 15 to the blower fan 50,
wherein the sterilization coupling portion 70 is arranged at the bell mouth 40.

13. The air conditioner 1 or 1a of claim 1, wherein the first frame 310 or 310a includes a first hole 311a and a second hole 311b spaced apart from the first hole 311a, and
the case 200 or 200a is connectable to the first hole 311a or the second hole 311b.

14. The air conditioner 1 or 1a of claim 10, wherein the holder 300 further includes a holder hinge 411 configured to allow the first frame 310 to rotate relative to the second frame 330.

15. The air conditioner 1 or 1a of claim 10, wherein the holder 300 or 300a further includes a cable receiving portion 320 configured to secure a cable that supplies power to the first LED 120a and the second LED 120b.
